**Europäisches Patentamt**

(19) **European Patent Office**

**Office européen des brevets**

(11) Veröffentlichungsnummer: **0 013 962**
**B1**

(12) ## EUROPÄISCHE PATENTSCHRIFT

(45) Veröffentlichungstag der Patentschrift:
**27.01.82**

(51) Int. Cl.³: **A 61 K 37/42,** A 23 L 1/30

(21) Anmeldenummer: **80100281.7**

(22) Anmeldetag: **21.01.80**

(54) **Aminosäuren und Mineralsalze enthaltende Infusionslösung.**

(30) Priorität: **23.01.79 CH 644/79**

(43) Veröffentlichungstag der Anmeldung:
**06.08.80 Patentblatt 80/16**

(45) Bekanntmachung des Hinweises auf die Patenterteilung:
**27.01.82 Patentblatt 82/4**

(84) Benannte Vertragsstaaten:
**AT BE DE FR GB IT NL SE**

(56) Entgegenhaltungen:
**DE-A1-2 657 381**
**DE-A1-2 658 984**
**DE-A1-2 700 043**
**DE-A1-2 759 133**

(73) Patentinhaber: **THERA Gesellschaft für Patentverwertung mbH, Schmidschneiderstrasse 15, D-8036 Herrsching (DE)**

(72) Erfinder: **Die Erfinder haben auf ihre Nennung verzichtet**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

BUNDESDRUCKEREI BERLIN

# 0 013 962

## Aminosäure und Mineralsäure enthaltende Infusionslösung

Es ist bekannt, daß der menschliche Organismus im Streßzustand, z. B. nach einer Operation oder Verletzung ungehemmt in allen Geweben Proteine aktiviert, um die darin vorhandenen Aminosäuren zu metabolisieren und zur Energieverwertung heranzuziehen. Dabei wird ein Teil der mobilisierten Aminosäuren bereits in den peripheren Geweben oxydativ abgebaut, weil den Geweben bei ihrem gesteigerten Umsatz in dieser Streßsituation Energiesubstrate fehlen und die Freisetzung der Fettsäuren aus den Fettdepots nur mit Verzögerung vonstatten geht. Da die Aminosäuren nicht nur aus den Geweben, z. B. dem Muskel stammen, sondern auch aus funktionell wichtigen Proteinen, können leicht Funktionsstörungen auftreten, die sich postoperativ, z. B. in einer vermehrten Infektanfälligkeit, bemerkbar machen.

Aus diesem Grund wird der Patient im allgemeinen nach einer Operation oder größeren Verletzung parenteral ernährt, d. h., es werden ihm Energiesubstrate intravenös zugeführt, um diesen Mangel an körpereigenen Substraten zu überwinden und so dem Organismus die wertvollen Aminosäuren einzusparen. Um die Aminosäuren in der Verbrennung der Zellen erfolgreich zu ersetzen, ist jedoch eine hohe Zufuhr von energiereichen Substraten, d. h. eine hohe kalorische parenterale Ernährung notwendig. Da im Streß gleichzeitig eine Glukoseverwertungsstörung besteht, erhält man bei alleinigem Ersatz der fehlenden Energiesubstrate durch Glukose relativ hohe Glukosespiegelwerte, wie sie z. B. bei einem Diabetiker auftreten, so daß bei dieser Behandlung hyperosmolare Zustände bzw. sogar hyperosmolare Komata auftreten können. Man hat deshalb die Glukose teilweise durch Zuckeraustauschstoffe, Fettemulsionen und durch Alkohol ersetzt. Leider haben alle diese energetisch geeigneten Substrate Nebenwirkungen, die sie für eine volle parenterale Ernährung nur mit Einschränkung brauchbar erscheinen lassen. So stören die Zuckeraustauschstoffe u. a. den Energiestoffwechsel in der Leber, während die Fettemulsionen die Infektresistenz des Körpers und der Alkohol den Fett- und Harnsäurestoffwechsel der Leber beeinflussen, so daß Leberschädigung, Infektionen und Gichtanfälle die Folgen sind.

Ein deutlicher Fortschritt wurde durch Zusatz von Kininen zu hochprozentigen glukosehaltigen Infusionslösungen erreicht. In der DE-OS 2 657 381 sind solche Infusionslösungen beschrieben, die je Liter 100 bis 300 g Glukose und 1 µg bis 1,6 mg Kinine enthalten sowie gegebenenfalls noch andere Infusionskomponenten. Dietze et al. haben in Klin. Wochenschrift, Bd. 55, 1357 (1972), und in Hoppe Seylers Z. Physiol. Chem., Bd. 358, S. 633 (1977), über den Einfluß von Bradykinin bzw. des Kallikrein-Kinin-Systems auf die Glukoseverwertung im Muskel berichtet. Auch Wicklmayr et al. haben eine Verbesserung der gestörten Glukoseverwertung durch Bradykinin bei Diabetikern und bei Patienten in postoperativem Streß festgestellt (vgl. Klinische Wochenschrift, Bd. 56, S. 1077 bis 1083, 1978). Diese Wirkung wird mit einer Stimulierung des Glukosetransports über die Zellmembran des Muskelgewebes erklärt, so daß die Glukose deutlich besser verwertet wird und keine hyperosmolaren Zustände eintreten können. Die Zelle kann dann auf andere Energiesubstrate vor allem auf Aminosäuren, Fett und Alkohol verzichten. Die aus den körpereigenen Proteinen stammenden, gegebenenfalls in den Infusionslösungen der DE-OS 2 657 381 neben der Glukose zusätzlich verabreichten Aminosäuren, stehen damit ganz der Schließung des Defektes an der Stelle der Körperverletzung (z. B. nach Operationen) zur Verfügung. Damit gelang es mit der bisherigen Infusionstherapie den Eiweißstoffwechsel dahingehend zu beeinflussen, daß man den Anteil der sonst aus den eiweißhaltigen Geweben mobilisierten Aminosäuren, der zur Energiegewinnung der Zelle herangezogen wurde, durch Glukose ersetzte. Die Unterbindung der Mobilisation der Aminosäuren aus den körpereigenen proteinhaltigen Geweben Leber, Muskeln, Nerven, Hirn war damit allerdings nicht gewährleistet, da der Organismus mit Hilfe der katabolen Hormone, die die anabole Wirkung des Insulins auf den Proteinstoffwechsel abschwächten, weiterhin Aminosäuren mobilisierte, um sie der Deckung des entstandenen Defektes des Organismus an der Stelle der Verletzung (z. B. nach Operationen) zur Verfügung zu stellen. Das hatte aber den Nachteil für den Patienten, daß trotz der bisherigen Infusionstherapie neben Nerven- und Muskeleiweiß auch besonders wichtige funktionelle Proteine des Bluts, die in speziellen Körperfunktionen ordnend mitwirken, z. B. in der Gerinnung, der Infektabwehr usw., ebenfalls mit eingeschmolzen wurden.

Es zeigte sich nun überraschend, daß dann, wenn bei der parenteralen Ernährung etwa 1000 Kalorien allein durch Aminosäuren ersetzt werden und die Aminosäuren mit Hilfe eines Aminosäuregemisches und einer bestimmten Menge Kinin ohne die früher für notwendig erachteten großen Mengen an zusätzlichen Energiesubstraten wie Glukose oder Zuckeralkohole verabreicht werden, der Eiweißstoffwechsel gegenüber der bisherigen Infusionstherapie noch besser beeinflußt werden konnte, so daß eine vollkommen ausgeglichene Stickstoffbilanz entsteht. Es ließ sich zeigen, daß diese Normalisierung des Aminosäurestoffwechsels in dieser katabolen Situation dadurch zustande kommt, daß neben dem Ersatz der körpereigenen Aminosäuren durch die exogen zugeführten Aminosäuren für die Verbrennung in den Zellen, durch das Kinin im Zusammenhang mit einem hohen Aminosäureangebot die körpereiweißerhaltende Wirkung des Insulins gegenüber den katabolen Hormonen verstärkt und dadurch die in den Körpergeweben vonstatten gehende Proteolyse unterbunden wird, wobei die aus dem Protein nicht mehr mobilisierten Aminosäuren durch die in der Aminosäureinfusion vorhandenen Aminosäuren ersetzt werden. Damit besteht zum erstenmal die Möglichkeit, dem Organismus die Eiweißdepots in Streßzuständen, z. B. postoperativ oder während Infektionen ohne eine bei vielen Patienten unerwünschte Glukosezufuhr durchführen zu müssen, zu erhalten. Die neuen glukosefreien Infusionslösungen zur kalorienarmen parenteralen Ernährung enthalten 10 bis 200 g essentielle und nicht essentielle Aminosäuren in üblicher Kombination sowie 50 bis 10 000 µg Kinine je Liter Infusionslösung. Als Kinin eignet sich auch bei diesen kalorienarmen Lösungen besonders das Nonapeptid Bradykinin sowie das Dekapeptid Kallidin. Es können auch verschiedene Kinine zusammen verwendet werden. Die erfindungsgemäßen Lösungen sind sehr stabil und lassen sich leicht hitzesterilisieren. Wegen des Fehlens der Glukose ist hierbei die Bildung von Nebenprodukten nicht zu befürchten.

Nach bisheriger Auffassung mußte zum Aufbau der körpereigenen Proteine und zur Hemmung der Proteolyse im postoperativen Streß eine sehr kalorienreiche Ernährung (»hochkalorische Ernährung«) durchgeführt werden. Wie jetzt erkannt wurde, wird auch ohne kalorische Steigerung der Ernährung die gewünschte Aminosäureverwertung durch die Gegenwart der Kinine in der Aminosäure-Infusionslösung erreicht. Ein wichtiger weiterer Fortschritt in der neuen parenteralen Ernährung besteht auch darin, daß keine hochmolaren, hyperosmotischen Lösungen verwendet werden, so daß die Aminosäure-Kinin-Lösungen, z. B. die 5- bis 10prozentigen Lösungen, auch in die periphere Vene verabreicht werden können. Bei den bekannten aminosäurehaltigen hochprozentigen Glukoselösungen mußte immer ein oftmals risikoreicher zentraler Venenkatheter in eine große, herznahe Vene eingeführt werden, durch die diese hyperosmotische Infusionslösung dem Organismus zugeführt werden konnte.

Die neue Art der parenteralen Ernährung bedeutet gerade für Patienten mit einer angeborenen oder erworbenen Glukoseverwertungsstörung, also bei juvenilen Diabetikern oder auch bei Erwachsenendiabetikern einen großen Fortschritt, da man den beim Diabetiker in den genannten Streßsituationen auftretenden außerordentlich hohen Proteinkatabolismus mit dieser neuen Lösung stoppen kann, ohne dabei die bisher bei der Infusion von hochprozentigen glukosehaltigen Lösungen immer noch mögliche Entgleisung des Glukosestoffwechsels befürchten zu müssen, zumal sehr hohe Dosen von Insulin wegen der Gefahr der Hyperoglykaemie in praxi nicht anwendbar sind.

Im allgemeinen kann der Patient je nach Bedarf etwa 250 bis 3000 ml der Aminosäurelösung je Tag erhalten, was etwa einer Gesamtmenge von 20 bis 200 g Aminosäure entspricht, wobei dem Patienten dann 0,6 bis 3 mg Kinin zugeführt wird, eine Menge, die innerhalb von 24 Stunden vom Körper gut toleriert wird.

Klinische Untersuchungen an einer Gruppe von 8 operierten Patienten zeigten eine deutliche Abnahme der Stickstoffausscheidung im Urin, wenn die aminosäurehaltige Infusionslösung erfindungsgemäß das Bradykinin enthält. Die Untersuchungen wurden hierbei in der 12. bis 72. postoperativen Stunde vorgenommen. Während dieser Zeit erhielten die Patienten zunächst eine kontinuierliche Tropfinfusion mit einer ca. 5prozentigen Aminosäurelösung gemäß nachfolgendem Beispiel (Infusionslösung A), die jedoch kein Bradykin enthält, und zwar in einer Menge von 2500 ml je 24 Stunden sowie noch Elektrolyte je nach klinischem Bedarf. Die durchschnittliche Stickstoffnettoausscheidung betrug 4,8 g N in dem Zeitraum von 12 bis 24 Stunden nach der Operation bzw. 5,6 g N in dem Zeitraum von 24 bis 36 Stunden nach der Operation.

Der Aminosäureinfusionslösung wurde dann während der Zeitspanne der 24. bis 48. postoperativen Stunde Bradykinin in einer Menge von 200 µg je Liter zugesetzt. Hierdurch trat ein Rückgang der Stickstoffexkretion in der 36. bis 60. postoperativen Stunde auf 0,6 g bzw. 1,0 g Stickstoff je 12 Stunden ein. Wurde dann die Bradykininzugabe abgesetzt und wieder die kininfreie Aminosäureinfusionslösung gegeben, stieg die Stickstoffausscheidung wieder auf 4,9 g je 12 Stunden, d. h. in der 60. bis 72. postoperativen Stunde, an. Diese Versuche lassen auf eine deutliche Verbesserung der Aminosäureverwertung im Sinne eines anabolen Effekts zum Aufbau körpereigenen Proteins schließen.

Auch in einer anderen Versuchsanordnung konnte gezeigt werden, daß durch die Gegenwart von Bradykinin die Aufnahme von Aminosäure in die Muskelzellen verbessert wird. Mißt man nämlich am menschlichen Unterarm die Aminosäurekonzentration im arteriellen Blutstrom und im venösen Blutstrom, so tritt bei kontinuierlicher Zugabe von Bradykinin in den arteriellen Blutstrom eine deutliche Abnahme der Aminosäurekonzentration im venösen Blut auf. Dies zeigt ebenfalls, daß unter dem Einfluß des Kinins ohne zusätzliche Glukosezufuhr eine gesteigerte Aminosäureaufnahme in der Muskelzelle eintritt, die als eine proteinanabole Wirkung zu interpretieren ist. Auch vermindert die erfindungsgemäße Infusionslösung den beim postoperativen Zustand normalerweise auftretenden gesteigerten Proteinabbau in den Zellen und im Blutserum der Patienten.

Für vier Infusionslösungen A, B, C und D mit einem etwa 5-, 10-, 20- und 2prozentigen Aminosäuregehalt werden die in der nachfolgenden Tabelle aufgeführten Substanzen in den angegebenen Mengen in üblichen Infusionsflaschen gefüllt, die nach der Hitzesterilisierung für Infusionszwecke unmittelbar verwendbar sind. Die Lösungen sind praktisch unbegrenzt stabil.

**0 013 962**

Beispiel

| | Infusionslösungen | | | |
|---|---|---|---|---|
| | B | A | C | D |
| Glutaminsäure | 19 g | 9,5 g | 38 g | 4,5 g |
| Alanin | 13 g | 6,5 g | 26 g | 3,0 g |
| Prolin | 15 g | 7,5 g | 30 g | 3,5 g |
| Glycocoll | 18 g | 9,0 g | 36 g | – |
| Arginin | 7,0 g | 3,5 g | 14 g | 2,0 g |
| Histidin | 2,0 g | 1,0 g | 4,0 g | 0,5 g |
| Valin | 3,0 g | 1,5 g | 6,0 g | 0,75 g |
| Tryptophan | 1,0 g | 0,5 g | 2,0 g | 0,25 g |
| Threonin | 2,2 g | 1,1 g | 4,0 g | 0,5 g |
| Pheny-alanin | 4,5 g | 2,0 g | 9,0 g | 1,1 g |
| Methionin | 4,0 g | 2,0 g | 8,0 g | 1,05 g |
| Lysin · HCl | 4,5 g | 2,0 g | 9,0 g | 1,25 g |
| Leucin | 4,5 g | 2,0 g | 9,0 g | 1,1 g |
| Isoleucin | 3,0 g | 2,0 g | 6,0 g | 0,8 g |
| NaOH | 1,5 g | 1,0 g | 3,0 g | 1,6 g |
| KOH | 1,6 g | 1,4 g | 1,9 g | 1,68 g |
| Magnesiumacetat · 4 $H_2O$ | 1,0 g | 1,0 g | 1,0 g | 1,07 g |
| Sorbit | – | – | – | 25 g |
| Xylit | – | – | – | 25 g |
| Aepfelsäure | 0,7 g | 0,4 g | 0,9 g | 3,0 g |
| Bradykinin | 200 µg | 200 µg | 400 µg | 350 µg |
| Aqua Dest. ad | 1000 ml | 1000 ml | 1000 ml | 1000 ml |

**Patentansprüche**

1. Aminosäuren und Mineralsalze enthaltende glukosefreie Infusionslösung zur kalorienarmen parenteralen Ernährung, dadurch gekennzeichnet, daß sie 10 bis 200 g essentielle und nichtessentielle Aminosäuren und Kinine in einer Menge von 50 bis 10 000 µg je Liter Lösung enthält.

2. Infusionslösung nach Anspruch 1, dadurch gekennzeichnet, daß sie Aminosäuren in einer Menge von 50 bis 100 g mit Kininen in einer Menge von 200 bis 1000 µm je Liter Lösung enthält.

3. Infusionslösung nach Anspruch 1 oder 2, dadurch gekennzeichnet, daß als Kinin Bradykinin vorhanden ist.

4. Infusionslösung nach Anspruch 1 oder 3, dadurch gekennzeichnet, daß sie 100 bis 5000 µg Bradykinin je Liter enthält.

**0 013 962**

## Claims

1. A glucose-free infusion solution containing amino acids and mineral salts for low-calory parenteral nutrition, characterized in that it contains of from 10 to 200 g essential and not essential amino acids and kinines in an amount of from 50 to 10 000 µg per liter solution.

2. The infusion solution as claimed in claim 1, characterized in that it contains amino acis in an amount of from 50 to 100 g and kinines in an amount of from 200 to 1000 µg per liter solution.

3. The infusion solution as claimed in claim 1 or claim 2, characterized in that bradykinin is used as kinin.

4. The infusion solution as claimed in claim 1 or claim 3, characterized in that it contains of from 100 to 5000 µg bradykinin per liter.

## Revendications

1. Solution de perfusion contenant des acides aminés et des sels minéraux, exempte de glucose, pour une alimentation pauvre en calories par voie parentérale, caractérisée en ce qu'elle contient entre 10 et 200 g d'amino-acides essentiels et non essentiels et entre 50 et 10 000 µg de kinines par litre de solution.

2. Solution de perfusion suivant la revendication 1, caractérisée en ce qu'elle contient entre 50 et 100 g d'amino-acides et entre 200 et 1000 µg de kinines par litre de solution.

3. Solution de perfusion suivant l'une des revendications 1 et 2, caractérisée en ce qu'elle contient comme kinine la bradykinine.

4. Solution de perfusion suivant l'une des revendications 1 et 3, caractérisée en ce qu'elle contient entre 100 et 5000 µg de bradykinine par litre.